# EUROPÄISCHE PATENTSCHRIFT

(11) **EP 2 745 831 B1**
(45) Veröffentlichungstag und Bekanntmachung des Hinweises auf die Patenterteilung: **12.08.2015**
(21) Anmeldenummer: 13193473.9
(22) Anmeldetag: 19.11.2013
(51) Int. Cl.: A61K 8/25, A61K 8/81, A61Q 17/04, A61K 8/58, A61K 8/06, A61K 8/04, A61K 8/60

(54) **Kosmetische Emulsion mit Silica Dimethyl Silylate**
Cosmetic emulsion with silica dimethyl silylate
Émulsion cosmétique comprenant du Silica Dimethyl Silylate

(30) Priorität: 21.12.2012 DE 102012224158
(43) Veröffentlichungstag der Anmeldung: 25.06.2014
(73) Patentinhaber: Beiersdorf AG, 20253 Hamburg (DE)
(72) Erfinder: Pasternak, Anja, 22085 Hamburg (DE); Eitrich, Anja, 25337 Kölln-Reisiek (DE); Fänger, Sabine, 22763 Hamburg (DE); Skubsch, Kerstin, 25497 Prisdorf (DE); Möllgaard, Svenja Lena, 22337 Hamburg (DE)

(56) Entgegenhaltungen:
- US-A1- 2010 202 984
- Anonymous: "Divine Rejuvenating Cream Product Description", MINTEL GNDP, 1. August 2007 (2007-08-01), Seiten 1-3, XP055110116, Internet Gefunden im Internet: URL:http://www.gnpd.com/sinatra/recordpage /752982/from_search/ayUf4xpkwg/ [gefunden am 2014-03-26]
- Anonymous: "Anti-Wrinkle SmoothCream Night Product Description", , 1. März 2010 (2010-03-01), Seiten 1-4, XP055110711, Internet Gefunden im Internet: URL:http://www.gnpd.com/sinatra/recordpage /1291690/from_search/F9oZscUGFa/ [gefunden am 2014-03-28]

## Beschreibung

Die vorliegende Erfindung betrifft eine kosmetische Emulsion enthaltend Polyglyceryl-3 Methylglucose Distearate, Silica Dimethyl Silylate und Acrylates/C10-30 Alkylacrylates Crosspolymer.

Der Wunsch, schön und attraktiv auszusehen, ist von Natur aus im Menschen verwurzelt. Auch wenn das Schönheitsideal im Laufe der Zeit Wandlungen erfahren hat, so ist das Streben nach einem makellosen Äußeren immer das Ziel der Menschen gewesen. Einen wesentlichen Anteil an einem schönen und attraktiven Äußeren hat dabei der Zustand und das Aussehen der Haut.

Hautpflegeprodukte bestehen in der Regel aus Emulsionen. Unter Emulsionen versteht man im Allgemeinen heterogene Systeme, die aus zwei nicht oder nur begrenzt miteinander mischbaren Flüssigkeiten bestehen, die üblicherweise als Phasen bezeichnet werden und bei denen eine der beiden Flüssigkeiten in Form feinster Tröpfchen in der anderen Flüssigkeit dispergiert ist. Äußerlich und mit bloßem Auge betrachtet erscheinen Emulsionen homogen.

Sind die beiden Flüssigkeiten Wasser und Öl und liegen Öltröpfchen fein verteilt in Wasser vor, so handelt es sich um eine Öl-in-Wasser-Emulsion (O/W-Emulsion, z. B. Milch). Der Grundcharakter einer O/W-Emulsion ist durch das Wasser geprägt. Bei einer Wasser-in-Öl-Emulsion (W/O-Emulsion, z. B. Butter) handelt es sich um das umgekehrte Prinzip, wobei der Grundcharakter hier durch das Öl bestimmt wird.

Die Vielzahl an kommerziell erhältlichen kosmetischen Emulsionen darf jedoch nicht darüber hinwegtäuschen, dass diese Zubereitungen des Standes der Technik eine Reihe von Nachteilen aufweisen.

Insbesondere wenn es sich um Emulsionen handelt, die mit einem Sprühapplikator (Pumpspender oder Aerosoldose) aus einem Vorratsbehältnis unter Druck direkt auf die Haut aufgetragen werden, eine Anwendungsform die insbesondere im Bereich der Sonnenschutzmittel verbreitet ist, tritt das Problem auf, dass die Zubereitungen einerseits Temperatur- und lagerstabil sein sollen und nicht zur vorzeitigen Phasentrennung neigen und andererseits hinreichend dünnflüssig sein müssen, um überhaupt versprühbar zu sein.

Ferner sollen die Zubereitungen zwar dünnflüssig sein, andererseits nach dem Auftrag auf die Haut wiederum nicht sofort zerfließen sondern vielmehr halbwegs ortsfest aufgesprüht werden können. Darüber hinaus soll die Emulsion möglichst gleichmäßig relativ breit verteilt in feinsten Tröpfchen auf die Haut aufgetragen werden und nicht, wie man es aus der Kindheit von Wasserpistolen her kennt, in einem dünnen Strahl auf der Haut auftreffen (sogenannter Jet-Stream Auftrag). Dieser Effekt ist keineswegs nur vom Sprühkopf und Sprühdruck abhängig, sondern beispielsweise auch von der Viskosität, Oberflächenspannung und anderen Zubereitungseigenschaften.

Das der Fachmann dann noch die eigentlichen kosmetischen Eigenschaften der Zubereitung natürlich nicht aus den Augen verlieren darf - bei Sonnenschutzmitteln kommen u.a. der Lichtschutzfaktor, die Wasserfestigkeit und/oder die Klebrigkeit und Einzugsgeschwindigkeit in die Haut in Betracht- vergrößert das Dilemma zusätzlich.

Es war daher die Aufgabe der vorliegenden Erfindung, die Nachteile des Standes der Technik zu beseitigen und eine sprühbare Emulsionsgrundlage -insbesondere für Sonnenschutzmittel- zu entwickeln, die einerseits hinreichend lager- und temperaturstabil ist und sich andererseits gleichmäßig relativ breit verteilt in feinsten Tröpfchen auf die Haut auftragen lässt ohne sofort auf der Haut zu zerfließen. Die Emulsionsgrundlage sollte auf der Haut nicht klebrig wirken, sondern sich leicht verteilen lassen und schnell in die Haut einziehen. Darüber hinaus sollte die Emulsionsgrundlage in einem weiten Anwendungsspektrum einsetzbar und insbesondere für unterschiedliche Lichtschutzfaktoren geeignet sein. Nicht zuletzt sollte es möglich sein, zur Erhöhung der Wasserfestigkeit der Zubereitung, die Emulsion mit Filmbildnern zu versetzen ohne dass die oben geschilderte Performance darunter leidet.

Überraschend gelöst wird die Aufgabe durch eine kosmetische Emulsion enthaltend
a) Polyglyceryl-3 Methylglucose Distearate,
b) Silica Dimethyl Silylate und
c) Acrylates/C10-30 Alkylacrylates Crosspolymer,
dadurch gekennzeichnet, dass die Zubereitung einen oder mehrere UV-Filter enthält, gewählt aus der Gruppe der Verbindungen 2-Phenylbenzimidazol-5-sulfonsäure und/oder deren Salze; Phenylen-1,4-bis-(2-benzimidazyl)-3,3'-5,5'-tetrasulfonsäuresalze; 1,4-di(2-oxo-10-Sulfo-3-bornylidenmethyl)-Benzol und dessen Salze; 4-(2-Oxo-3-bornylidenmethyl)benzolsulfonsäuresalze ; 2-Methyl-5-(2-oxo-3-bornylidenmethyl)sulfonsäuresalze; 2,2'-Methylen-bis-(6-(2H-benzotriazol-2-yl)-4-(1,1,3,3-tetramethylbutyl)-phenol); 2-(2H-benzotriazol-2-yl)-4-methyl-6-[2-methyl-3-[1,3,3,3-tetramethyl-1-[(trimethylsilyl)oxy]disiloxanyl]propyl]-phenol; 3-(4-Methylbenzyliden)campher; 3-Benzylidencampher; Ethylhexylsalicylat; Terephthalidendicamphersulfonsäure; 2-Ethylhexyl-2-cyano-3,3-diphenylacrylat; 4-(Dimethylamino)-benzoesaure(2-ethylhexyl)ester; 4-(Dimethylamino)benzoesäure-amylester; 4-Methoxybenzalmalon-säuredi(2-ethylhexyl)ester; 4-Methoxyzimtsäure(2-ethylhexyl)ester; 4-Methoxyzimtsäureisoamylester: 2-Hydroxy-4-methoxybenzophenon, 2-Hydroxy-4-methoxy-4'-methylbenzophenon; 2,2'-Dihydroxy-4-methoxybenzophenon; Homomenthylsalicylat; 2-Ethylhexyl-2-hydroxybenzoat; Dimethicodiethylbenzalmalonat; 3-(4-(2,2-bis Ethoxycarbonylvinyl)-phenoxy)propenyl)-methoxysiloxan / Dimethylsiloxan - Copolymer; 4-(tert.-Butyl)-4'-methoxydibenzoylmethan; 2-(4'-Diethylamino-2'-hydoxybenzoyl)-benzoesäurehexylester, Dioctylbutylamidotriazon (INCI: Diethylhexyl-Butamidotriazone); 2,4-bis-[5-1(dimethylpropyl)benzoxazol-2-yl-(4-phenyl)imino]-6-(2-ethylhexyl)-imino-1,3,5-triazin mit der (CAS Nr. 288254-16-0); 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Ethoxyphenyl Triazin); 4,4',4"-(1,3,5-Triazin-2,4,6-triyltriimino)-tris-benzoësäure-tris(2-ethylhexylester) (auch: 2,4,6-Tris-[anilino-(p-carbo-2'-ethyl-1'-hexyloxy)]-1,3,5-triazin (INCI: Ethylhexyl Triazone); 2,4,6-Tribiphenyl-4-yl-1,3,5-triazin; Merocyanine.

Zwar kennt der Fachmann die DE 19645318, DE 19645319, DE 19645320, DE 102004052833, DE 102008021631 und DE 102006006864, doch konnten diese Schriften ebenso wenig den Weg zur vorliegenden Erfindung weisen wie die US 2010/0202984 oder die Einträge in der Mintel-Datenbank der GNPD mit den Record IDs 752982 (Divine Rejuvenating Gream) und 1291690 (Anti-Wrinkle SmoothCream).

Es ist erfindungsgemäß vorteilhaft, wenn die Zubereitung einen oder mehrere UV-Filter enthält. Insbesondere ist es erfindungsgemäß bevorzugt, wenn die erfindungsgemäße Emulsion ein Sonnenschutzmittel ist.

Erfindungsgemäß bevorzugt handelt es sich bei der erfindungsgemäßen Emulsion um eine O/W-Emulsion.

Es ist erfindungsgemäß vorteilhaft, wenn die kosmetische Emulsion Polyglyceryl-3 Methylglucose Distearate in einer Konzentration von 0,05 bis 5 Gewichts-%, bezogen auf das Gesamtgewicht der Zubereitung, enthält.

Es ist erfindungsgemäß bevorzugt, wenn die kosmetische Emulsion Polyglyceryl-3 Methylglucose Distearate in einer Konzentration von 0,1 bis 1 Gewichts-%, bezogen auf das Gesamtgewicht der Zubereitung, enthält.

Der Emulgator Polyglyceryl-3 Methylglucose Distearate kann bei der Firma Evonik unter dem Handelsnamen Tegocare 450 käuflich erworben werden.

Es ist erfindungsgemäß vorteilhaft, wenn die erfindungsgemäße Zubereitung Silica Dimethyl Silylate in einer Konzentration von 0,05 bis 5 Gewichts-%, bezogen auf das Gesamtgewicht der Zubereitung, enthält.

Es ist erfindungsgemäß bevorzugt, wenn die erfindungsgemäße Zubereitung Silica Dimethyl Silylate in einer Konzentration von 0,1 bis 1 Gewichts-%, bezogen auf das Gesamtgewicht der Zubereitung, enthält.

Silica Dimethyl Silylate kann beispielsweise bei der Firma Evonik unter dem Handelsnamen Aerosil R 972 oder Aerosil R 974 käuflich erworben werden.

Es ist erfindungsgemäß vorteilhaft, wenn die erfindungsgemäße Emulsion Acrylates/C10-30 Alkylacrylates Crosspolymer in einer Konzentration von 0,05 bis 1,5 Gewichts-%, bezogen auf das Gesamtgewicht der Zubereitung, enthält.

Es ist erfindungsgemäß bevorzugt, wenn die erfindungsgemäße Emulsion Acrylates/C10-30 Alkylacrylates Crosspolymer in einer Konzentration von 0,1 bis 0,8 Gewichts-%, bezogen auf das Gesamtgewicht der Zubereitung, enthält.

Acrylates/C10-30 Alkylacrylates Crosspolymer kann beispielsweise bei der Firma Lubrizol unter den Handelsnamen Pemulen TR-1 und Pemulen TR-2 käuflich erworben werden.

Acrylates/C10-30 Alkylacrylates Crosspolymer ist dabei eindeutig zu unterscheiden von dem ebenfalls bekannten Poly C10-30 Alkyl Acrylate, welches nicht quervernetzt ist.

Erfindungsgemäß bevorzugt ist es dabei wenn die erfindungsgemäße Emulsion einen oder mehrere UV-Filter gewählt aus der Gruppe 2-Ethylhexyl-2-cyano-3,3-diphenylacrylat, Ethylhexylsalicylat und/oder 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazin) enthält. Es ist erfindungsgemäß besonders bevorzugt, wenn die erfindungsgemäße Emulsion oder 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazin) enthält.

Es ist darüber hinaus erfindungsgemäß von Vorteil, wenn die Zubereitung Xanthangummi enthält.

In einem solchen Falle ist es erfindungsgemäß von Vorteil, wenn die Zubereitung Xanthangummi in einer Konzentration von 0,05 bis 0,5 Gewichts-%, bezogen auf das Gesamtgewicht der Zubereitung, enthält.

Erfindungsgemäß vorteilhafte Ausführungsformen sind dadurch gekennzeichnet, dass die Zubereitung einen oder mehrere Wirkstoffe gewählt aus der Gruppe der Verbindungen Gylcyrrhetinsäure, Harnstoff, Arctiin, alpha-Liponsäure, Folsäure, Phytoen, D-Biotin, Coenzym Q10, alpha-Glucosylrutin, Carnitin, Carnosin, Coffein, natürliche und/oder synthetische Isoflavonoide, Glycerylglucose, Kreatin, Kreatinin, Taurin, Tocopherol, Tocopherolacetat, β-Alanin und/oder Licochalcon A, enthält.

Es ist erfindungsgemäß vorteilhaft, wenn die Zubereitung Propylenglycol, Butylenglycol, 2-Methylpropan-1,3-diol, 1,2-Pentandiol, 1,2-Hexandiol, 1,2-Octandiol und/oder 1,2-Decandiol enthält.

Es ist erfindungsgemäß von Vorteil, wenn die Zubereitung Phenoxyethanol und/oder Methylisothiazolinon enthält.

Erfindungsgemäß vorteilhafe Ausführungsformen zeichnen sich dadurch aus, dass die Zubereitung eine Viskosität von 200 bis 2500 mPas bei einer Temperatur von 25 °C gemessen mit Rheomat 123 von proRheo mit Spindel 1 (Messbereich 300-10000 mPas).

Die erfindungsgemäßen kosmetischen Zubereitungen können ferner vorteilhaft, wenngleich nicht zwingend, Füllstoffe enthalten, welche z. B. die sensorischen und kosmetischen Eigenschaften der Formulierungen weiter verbessern und beispielsweise ein samtiges oder seidiges Hautgefühl hervorrufen oder verstärken. Vorteilhafte Füllstoffe im Sinne der vorliegenden Erfindung sind Stärke und Stärkederivate (wie z. B. Tapiocastärke, Distärkephosphat, Aluminium- bzw. Natrium-Stärke Octenylsuccinat und dergleichen), Pigmente, die weder hauptsächlich UV-Filter- noch färbende Wirkung haben (wie z. B. Bornitrid etc.) und/oder Aerosile^{®} (CAS-Nr. 7631-86-9), Talkum, Lauroyl Lysine und Acrylonitrilemethacrylonitrile-methyl-methacrylate.

Erfindungsgemäß bevorzugt ist es, wenn die Zubereitung Füllstoffe, insbesondere Polyethylen, Nylon, natürliche oder modifizierte Stärken wie Tapiokastärke und/oder Silikate wie beispielsweise Talkum enthält.

Die Wasserphase der erfindungsgemäßen Zubereitungen kann vorteilhaft übliche kosmetische Hilfsstoffe enthalten, wie beispielsweise Alkohole, insbesondere solche niedriger C-Zahl wie Isopropanol, Diole oder Polyole niedriger C-Zahl sowie deren Ether, vorzugsweise Propylenglykol, 2-Methylpropan-1,3-diol, Pentan-1,2-diol, Hexan-1,2-diol, Octan-1,2-diol, Decan-1,2-diol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, Schaumstabilisatoren, Elektrolyte, etc..

Die Ölphase der erfindungsgemäßen Zubereitung wird vorteilhaft gewählt aus der Gruppe der polaren Öle, beispielsweise aus der Gruppe der Lecithine und der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, wie z. B. Cocoglycerid, Olivenöl, Sonnenblumenöl, Jojobaöl, Sojaöl, Erdnußöl, Rapsöl, Mandelöl, Palmöl, Kokosöl, Rizinusöl, Weizenkeimöl, Traubenkernöl, Distelöl, Nachtkerzenöl, Macadamianußöl und dergleichen mehr.

Erfindungsgemäß vorteilhaft sind ferner z. B. natürliche Wachse tierischen und pflanzlichen Ursprungs, wie beispielsweise Bienenwachs und andere Insektenwachse sowie Beerenwachs, Sheabutter und/oder Lanolin (Wollwachs).

Weitere vorteilhafte polare Ölkomponenten können im Sinne der vorliegenden Erfindung ferner gewählt werden aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen sowie aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Phenethylbenzoat, 2-Phenylethylbenzoat, Isopropyl Lauroyl Sarkosinat, Phenyl Trimethicon, Cyclomethicon, Dibutyladipat, Octylpalmitat, Octylcocoat, Octylisostearat, Octyldodeceylmyristat, Octyldodekanol, Cetearylisononanoat, Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Stearylheptanoat, Oleyloleat, Oleylerucat, Erucyloleat, Erucylerucat, Tridecylstearat, Tridecyltrimellitat, sowie synthetische, halbsynthetische und natürliche Gemische solcher Ester, wie z. B. Jojobaöl.

Ferner kann die Ölphase vorteilhaft gewählt werden aus der Gruppe der Dialkylether und Dialkylcarbonate, vorteilhaft sind z. B. Dicaprylylether (*Cetiol OE*) und/oder Dicaprylylcarbonat, beispielsweise das unter der Handelsbezeichnung *Cetiol CC* bei der Fa. Cognis erhältliche.

Es ist ferner vorteilhaft, das oder die Ölkomponenten aus der Gruppe Isoeikosan, Neopentylglykoldiheptanoat, Propylenglykoldicaprylat/dicaprat, Caprylic/Capric/Diglycerylsuccinat, Butylenglykol Dicaprylat/Dicaprat, C₁₂₋₁₃-Alkyllactat, Di-C₁₂₋₁₃-Alkyltartrat, Triisostearin, Dipentaerythrityl Hexacaprylat/Hexacaprat, Propylenglykolmonoisostearat, Tricaprylin, Dimethylisosorbid. Es ist insbesondere vorteilhaft, wenn die Ölphase der erfindungsgemäßen Formulierungen einen Gehalt an C₁₂₋₁₅-Alkylbenzoat aufweist oder vollständig aus diesem besteht.

Vorteilhafte Ölkomponenten sind ferner z. B. Butyloctylsalicylat (beispielsweise das unter der Handelsbezeichnung *Hallbrite BHB* bei der Fa. CP Hall erhältliche), Tridecylsalicylat (welches unter der Handelsbezeichnung Cosmacol ESI bei der Fa. Sasol erhältlich ist), C12-C15 Alkylsalicylat (unter der Handelsbezeichnung Dermol NS bei der Fa. Alzo erhältlich), Hexadecylbenzoat und Butyloctylbenzoat und Gemische davon (*Hallstar AB*).

Auch beliebige Abmischungen solcher Öl- und Wachskomponenten sind vorteilhaft im Sinne der vorliegenden Erfindung einzusetzen.

Ferner kann die Ölphase ebenfalls vorteilhaft auch unpolare Öle enthalten, beispielsweise solche, welche gewählt werden aus der Gruppe der verzweigten und unverzweigten Kohlenwasserstoffe und -wachse, insbesondere Mineralöl, Vaseline (Petrolatum), Paraffinöl, Squalan und Squalen, Polyolefine, hydrogenierte Polyisobutene, C13-16 Isoparaffin und Isohexadecan. Unter den Polyolefinen sind Polydecene die bevorzugten Substanzen.

Erfindungsgemäß vorteilhaft enthält die erfindungsgemäße Zubereitung Filmbildner. Filmbildner im Sinne der vorliegenden Erfindung sind Stoffe unterschiedlicher Zusammensetzung, die durch die folgende Eigenschaft charakterisiert sind: Löst man einen Filmbildner in Wasser oder anderen geeigneten Lösungsmitteln und trägt die Lösung dann auf die Haut auf, so bildet er nach dem Verdunsten des Lösemittels einen Film aus, der im wesentlichen dazu dient, die Lichtfilter auf der Haut zu fixieren und so die Wasserfestigkeit des Produktes zu steigern.

Es ist insbesondere von Vorteil, die Filmbildner aus der Gruppe der Polymere auf Basis von Polyvinylpyrrolidon (PVP) 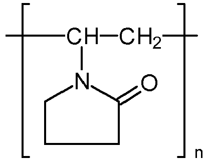 zu wählen. Besonders bevorzugt sind Copolymere des Polyvinylpyrrolidons, beispielsweise das PVP Hexadecen Copolymer und das PVP Eicosen Copolymer, welche unter den Handelsbezeichnungen Antaron V216 und Antaron V220 bei der GAF Chemicals Cooperation erhältlich sind.

Ebenfalls vorteilhaft sind weitere polymere Filmbildner, wie beispielsweise Natriumpolystryrensulfonat, welches unter der Handelsbezeichnung Flexan 130 bei der National Starch and Chemical Corp. erhältlich ist, und/oder Polyisobuten, erhältlich bei Rewo unter der Handelsbezeichnung Rewopal PIB1000. Weitere geeignete Polymere sind z.B. Polyacrylamide (Seppigel 305), Polyvinylalkohole, PVP, PVP / VA Copolymere, Polyglycole, Acrylat/Octylacralymid Copolymer (Dermacryl 79). Ebenfalls vorteilhaft ist die Verwendung von Hydriertem Rizinusöl Dimerdilinoleat (CAS 646054-62-8, INCI Hydrogenated Castor Oil Dimer Dilinoleate), das bei der Firma Kokyu Alcohol Kogyo unter dem Namen Risocast DA-H erworben werden kann oder aber auch PPG-3 Benzylethermyristat (CAS 403517-45-3), das unter dem Handelsnamen Crodamol STS bei der Firma Croda Chemicals erworben werden kann.

Erfindungsgemäß ist ein Pumpspender enthaltend eine erfindungsgemäße Emulsion. Erfindungsgemäß ist auch eine Aerosoldose enthaltend eine erfindungsgemäße Emulsion.

### Vergleichsversuch

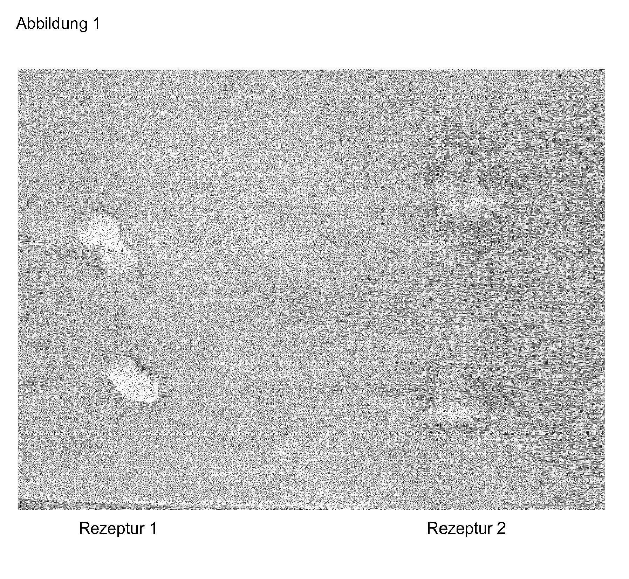

Mit dem folgenden Versuch konnte der erfindungsgemäße Effekt beispielhaft belegt werden:
Es wurden die folgenden Rezepturen hergestellt und mit einem Pumpspender bei 20 °C aus einer Entfernung von 30 cm, rechtwinklig auf eine Wand aus Handtüchern von SCOTT (LxB: 304000 mm x 200 mm; 1-lagig; blau) gesprüht. Das Sprühbild ist in Abbildung 1 abgebildet (siehe Anhang) Links ist die Rezeptur 1 zu sehen, rechts die Rezeptur 2.

| **Inhaltsstoffe** | **Rezeptur 1** | **Rezeptur 2** |
|---|---|---|
| **Silica Dimethyl Silylate** | **0** | **0,5** |
| Emulgator-und Verdickersystem | 0,7 | 0,7 |
| UV-Filter- und Öl-Phase | 28,5 | 28,5 |
| Moisturizer-Phase | 6 | 6 |
| Additive | 2,46 | 2,46 |
| Konservierungssystem | 4,9 | 4,9 |
| Parfum | 0,3 | 0,3 |
| Aqua | 57,14 | 56,64 |

**Fazit:** Die erfindungsgemäße Zubereitung Rezeptur 2 mit Silica Dimethyl Silylate führt zu einem deutlich gleichmäßigeren und großflächigeren Sprühbild als Rezeptur 1. Darüber hinaus sind die Tropfen des Sprühbildes bei Rezeptur 2 deutlich feiner und kleiner (nebelartiger), während bei Rezeptur 1 ein einziger großer Klecks entsteht.

### Beispiele

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

| **INCI** | **1** | **2** | **3** | **4** | **5** |
|---|---|---|---|---|---|
| Polyglyceryl-3 Methylglucose Distearate | 0,05 | 0,2 | 0,45 | 0,1 | 1 |
| Silica Dimethyl Silyate | 1 | 0,5 | 0,5 | 2 | 5 |
| Acrylate/C10-30 Alkyl Acrylate Crosspolymer | 0,75 | 0,4 | 0,2 | 0,05 | 1 |
| Xanthan Gum | 0,1 | 0,15 | 0,05 | 0,4 | 0,5 |
| Homosalate | 8 | 5 | 7 | 3 | 4,5 |
| Octocrylen | 6 | 7 | 8 | 0 | 0 |
| Bis-Ethylhexyloxyphenol Methoxyphenyl Triazine | 1 | 2,5 | 0 | 0 | 0 |
| Butyl Methoxydibenzoylmethane | 1,5 | 3,5 | 4,5 | 1,5 | 2,25 |
| Titanium Dioxide | 3 | 0 | 0 | 0,7 | 1 |
| Phenylbenzimidazole Sulfonic Acid | 0,5 | 1,5 | 0 | 2 | 2 |
| Ethylhexyl Salicylate | 4 | 4,5 | 4,5 | 4 | 2,25 |
| VP/Hexadecene Copolymer | 0,5 | 0 | 1 | 0,2 | 0 |
| Trisodium EDTA | 0,2 | 0,2 | 0,2 | 0,2 | 0,2 |
| Phenoxyethanol | 0,5 | 0,5 | 0,6 | 0,6 | 0,6 |
| Ethylparaben | 0,1 | 0,1 | 0,2 | 0,2 | 0,15 |
| Methylparaben | 0,3 | 0,3 | 0,3 | 0 | 0,3 |
| Alcohol Denat. | 4 | 4 | 4 | 4 | 4 |
| 1,2-Pentandiol | 0 | 0,5 | 0 | 0 | 0 |
| Glycerin | 5 | 5 | 10 | 2 | 10 |
| Glyceryl Glucoside | 0 | 0 | 2 | 5 | 3 |
| Panthenol | 2 | 1 | 0,5 | 0 | 0 |
| Tocopheryl Acetate | 0,05 | 1 | 5 | 0 | 0 |
| Lanolin | 0 | 0 | 0 | 0,1 | 0 |
| Butylene Glycol Dicaprylate/Dicaprate | 4 | 2 | 0 | 5 | 0 |
| C12-15 Alkyl Benzoate | 0 | 0 | 4 | 5 | 0 |
| Cetearyl Alcohol | 0 | 0 | 0 | 0,5 | 0,2 |
| Myristyl Myristate | 0 | 0 | 0,5 | 0 | 0 |
| Dimethicone | 0 | 0 | 0,8 | 0,8 | 0,8 |
| Caprylic/Capric Triglyceride | 0 | 0 | 0 | 0 | 7 |
| Butyloctylsalicylate | 0 | 0 | 0 | 0 | 4 |
| CI 42090 | 0,002 | 0 | 0 | 0 | 0 |
| Parfum | 0,3 | 0,4 | 0,4 | 0,4 | 0,4 |
| Sodium Hydoxide | Adjust to pH 7 | | | | |
| Aqua | Ad 100 | | | | |
| Polyglyceryl-3 Methylglucose Distearate | 1,5 | 1,7 | 0,15 | 4 | 5 |
| Silica Dimethyl Silyate | 3 | 4 | 0,3 | 0,9 | 0,05 |
| Acrylate/C10-30 Alkyl Acrylate Crosspolymer | 0,3 | 0,3 | 0,1 | 0,1 | 0,05 |
| Xanthan Gum | 1 | 0,05 | 0,1 | 0,1 | 0,05 |
| Sodium Carbomer | 0 | 0 | 0 | 0 | 0,1 |
| Microcristalline Cellulose + Cellulose Gum | 0 | 0 | 0 | 3 | 0 |
| Ethylhexyl Methoxycinnamate | 0 | 0 | 7 | 0 | 0,5 |
| Homosalate | 8 | 4 | 0 | 9 | 8 |
| Octocrylen | 3 | 8 | 3 | 9 | 4,5 |
| Bis-Ethylhexyloxyphenol Methoxyphenyl Triazine | 0 | 0 | 3 | 0 | 0 |
| Butyl Methoxydibenzoylmethane | 1,5 | 2,5 | 1,5 | 3 | 3,25 |
| Diethylhexyl Butamino Triazone | 0 | 0 | 0 | 0 | 1 |
| Titanium Dioxide | 0,7 | 0 | 2 | 0 | 1 |
| Phenylbenzimidazole Sulfonic Acid | 0 | 1 | 1,5 | 0 | 0 |
| Ethylhexyl Salicylate | 1,5 | 3 | 0 | 0 | 0 |
| VP/Hexadecene Copolymer | 0 | 1,5 | 0,5 | 0 | 0,3 |
| Trisodium EDTA | 0,2 | 0,2 | 0,2 | 0,2 | 0,2 |
| Phenoxyethanol | 0,6 | 0 | 0 | 0,4 | 0 |
| Ethylparaben | 0,05 | 0 | 0 | 0 | 0 |
| Methylparaben | 0,3 | 0 | 0 | 0,3 | 0 |
| Methylpropanediol | 0 | 1 | 2 | 0 | 1 |
| Alcohol Denat. | 4 | 6 | 8 | 0 | 6 |
| Ethylhexylglycerin | 0 | 0,5 | 0,5 | 0 | 0,5 |
| C18-36 Acid Triglyceride | 0 | 0 | 0,5 | 0 | 0 |
| Tapiokastärke | 0 | 0 | 0 | 0 | 2 |
| 1,2-Pentandiol | 0 | 0,5 | 0 | 0 | 0 |
| 1,2-Hexandiol | 0 | 0 | 0 | 0 | 0,5 |
| Glycerin | 5 | 5 | 8 | 4 | 2 |
| Glyceryl Glucoside | 0,1 | 1 | 0,5 | 6 | 11 |
| Tocopheryl Acetate | 0 | 2 | 1 | 0 | 4 |
| Aloe Barbadensis Leaf Juice | 0 | 0,5 | 0 | 0 | 0,5 |
| Glycyrrhetinsäure | 0,1 | 0 | 0 | 0 | 0 |
| Sonnenblumenöl | 0 | 0 | 0 | 2 | 0 |
| Butylene Glycol Dicaprylate/Dicaprate | 0 | 0 | 4 | 0 | 6 |
| C12-15 Alkyl Benzoate | 0 | 5 | 0 | 2 | 0 |
| Cetearyl Alcohol | 0,1 | 0 | 0 | 0 | 0 |
| Dimethicone | 0 | 2 | 0 | 0 | 0 |
| Caprylic/Capric Triglyceride | 5 | 0 | 0 | 0 | 0 |
| Dicaprylylether | 4 | 3 | 0 | 0 | 0 |
| C13-16 Isoparaffin | 0 | 0 | 2 | 0 | 0 |
| CI 42090 | 0 | 0 | 0 | 0 | 0,002 |
| Parfum | 0,4 | 0,4 | 0,4 | 0,3 | 0,3 |
| Sodium Hydoxide | Adjust to pH 7 | | | | |
| Aqua | Ad 100 | | | | |

## Patentansprüche

1. Kosmetische Emulsion enthaltend
a) Polyglyceryl-3 Methylglucose Distearate,
b) Silica Dimethyl Silylate und
c) Acrylates/C10-30 Alkylacrylates Crosspolymer,
**dadurch gekennzeichnet, dass** die Zubereitung einen oder mehrere UV-Filter enthält, gewählt aus der Gruppe der Verbindungen 2-Phenylbenzimidazol-5-sulfonsäure und/oder deren Salze; Phenylen-1,4-bis-(2-benzimidazyl)-3,3'-5,5'-tetrasulfonsäuresalze; 1,4-di(2-oxo-10-Sulfo-3-bornylidenmethyl)-Benzol und dessen Salze; 4-(2-Oxo-3-bornylidenmethyl)-benzolsulfonsäuresalze; 2-Methyl-5-(2-oxo-3-bornylidenmethyl)sulfonsäuresalze; 2,2'-Methylen-bis-(6-(2H-benzotriazol-2-yl)-4-(1,1,3,3-tetramethylbutyl)-phenol); 2-(2H-benzotriazol-2-yl)-4-methyl-6-[2-methyl-3-[1,3,3,3-tetramethyl-1-[(trimethylsilyl)oxy]disiloxanyl]propyl]-phenol; 3-(4-Methylbenzyliden)campher; 3-Benzylidencampher; Ethylhexylsalicylat; Terephthalidendicamphersulfonsäure; 2-Ethylhexyl-2-cyano-3,3-diphenylacrylat; 4-(Dimethylamino)-benzoesäure(2-ethylhexyl)ester 4-(Dimethylamino)benzoesäure-amylester; 4-Methoxybenzalmalon-säuredi(2-ethylhexyl)-ester; 4-Methoxyzimtsäure(2-ethylhexyl)ester; 4-Methoxyzimtsäureisoamylester; 2-Hydroxy-4-methoxybenzophenon, 2-Hydroxy-4-methoxy-4'-methylbenzophenon; 2,2'-Dihydroxy-4-methoxybenzophenon; Homomenthylsalicylat; 2-Ethylhexyl-2-hydroxybenzoat; Dimethicodiethylbenzalmalonat; 3-(4-(2,2-bis Ethoxycarbonylvinyl)-phenoxy)propenyl)-methoxysiloxan / Dimethylsiloxan - Copolymer; 4-(tert.-Butyl)-4'-methoxydibenzoylmethan; 2-(4'-Diethylamino-2'-hydoxybenzoyl)-benzoesäurehexylester; Dioctylbutylamidotriazon (INCI: Diethylhexyl-Butamidotriazone); 2,4-bis-[5-1(dimethylpropyl)benzoxazol-2-yl-(4-phenyl)-imino]-6-(2-ethylhexyl)-imino-1,3,5-triazin mit der (CAS Nr. 288254-16-0); 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazin); 4,4',4"-(1,3,5-Triazin-2,4,6-triyltriimino)-tris-benzoësäure-tris(2-ethylhexylester) (auch: 2,4,6-Tris-[anilino-(p-carbo-2'-ethyl-1'-hexyloxy)]-1,3,5-triazin (INCI: Ethylhexyl Triazone); 2,4,6-Tribiphenyl-4-yl-1,3,5-triazin; Merocyanine..

2. Kosmetische Emulsion nach einem der vorhergehenden Ansprüche, **dadurch gekennzeichnet, dass** die Zubereitung Xanthangummi enthält.

3. Kosmetische Emulsion nach einem der vorhergehenden Ansprüche, **dadurch gekennzeichnet, dass** die Zubereitung Polyglyceryl-3 Methylglucose Distearate in einer Konzentration von 0,05 bis 5 Gewichts-%, bezogen auf das Gesamtgewicht der Zubereitung, enthält.

4. Kosmetische Emulsion nach einem der vorhergehenden Ansprüche, **dadurch gekennzeichnet, dass** die Zubereitung Silica Dimethyl Silylate in einer Konzentration von 0,05 bis 5 Gewichts-%, bezogen auf das Gesamtgewicht der Zubereitung, enthält.

5. Kosmetische Emulsion nach einem der vorhergehenden Ansprüche, **dadurch gekennzeichnet, dass** die Zubereitung Acrylates/C10-30 Alkylacrylates Crosspolymer in einer Konzentration von 0,05 bis 1,5 Gewichts-%, bezogen auf das Gesamtgewicht der Zubereitung, enthält.

6. Kosmetische Emulsion nach einem der vorhergehenden Ansprüche, **dadurch gekennzeichnet, dass** die Zubereitung Xanthangummi in einer Konzentration von 0,05 bis 0,5 Gewichts-%, bezogen auf das Gesamtgewicht der Zubereitung, enthält.

7. Kosmetische Emulsion nach einem der vorhergehenden Ansprüche, **dadurch gekennzeichnet, dass** die Zubereitung einen oder mehrere Wirkstoffe gewählt aus der Gruppe der Verbindungen Gylcyrrhetinsäure, Harnstoff, Arctiin, alpha-Liponsäure, Folsäure, Phytoen, D-Biotin, Coenzym Q10, alpha-Glucosylrutin, Carnitin, Carnosin, Coffein, natürliche und/oder synthetische Isoflavonoide, Glycerylglucose, Kreatin, Kreatinin, Taurin, Tocopherol, Tocopherolacetat, ß-Alanin und/oder Licochalcon A, enthält.

8. Kosmetische Emulsion nach einem der vorhergehenden Ansprüche, **dadurch gekennzeichnet, dass** die Zubereitung Propylenglycol, Butylenglycol, 2-Methylpropan-1,3-diol, 1,2-Pentandiol, 1,2-Hexandiol, 1,2-Octandiol und/oder 1,2-Decandiol enthält.

9. Kosmetische Emulsion nach einem der vorhergehenden Ansprüche, **dadurch gekennzeichnet, dass** die Zubereitung Phenoxyethanol und/oder Methylisothiazolinon enthält.

10. Kosmetische Emulsion nach einem der vorhergehenden Ansprüche, **dadurch gekennzeichnet, dass** die Zubereitung eine Viskosität von 200 bis 2500 mPas bei einer Temperatur von 25 °C gemessen mit Rheomat 123 von proRheo mit Spindel 1 (Messbereich 300-10000 mPas) aufweist.

11. Pumpspender enthaltend eine Emulsion nach einem der Ansprüche 1 bis 12.

12. Aerosoldose enthaltend eine Emulsion nach einem der Ansprüche 1 bis 12.

## Claims

1. Cosmetic emulsion comprising
a) polyglyceryl-3 methylglucose distearate,
b) silica dimethyl silylate and
c) acrylates/C10-30 alkyl acrylates crosspolymer,
**characterized in that** the preparation comprises one or more UV filters selected from the group of the compounds 2-phenylbenzimidazole-5-sulphonic acid and/or salts thereof; phenylene-1,4-bis(2-benzimidazyl)-3,3'-5,5'-tetrasulphonic acid salts; 1,4-di(2-oxo-10-sulpho-3-bomylidenemethyl)benzene and salts thereof; 4-(2-oxo-3-bornylidenemethyl)benzenesulphonic acid salts; 2-methyl-5-(2"'oxo-3-bornylidenemethyl)sulphonic acid salts; 2,2'-methylenebis(6-(2H-benzotriazol-2-yl)-4-(1,1,3,3-tetramethylbutyl)phenol); 2-(2H-benzotriazol-2-yl)-4-methyl-6-[2-methyl-3-[1,3,3,3-tetramethyl-1-[(trimethylsilyl)oxy]-disiloxanyl]propyl]phenol; 3-(4-methylbenzylidene)camphor; 3-benzylidenecamphor; ethyl-hexyl salicylate; terephthalidenedicarnphorsulphonic acid; 2-ethylhexyl 2-cyano-3,3-diphenylacrylate; 4-(dimethylamino)benzoic acid (2-ethylhexyl) ester; 4-(dimethylamino)benzoic acid amyl ester; 4-methoxybenzalmalonic acid di(2-ethylhexyl) ester; 4-methoxycinnamic acid (2-ethylhexyl) ester; 4-methoxycinnamic acid isoamyl ester; 2-hydroxy-4-methoxybenzophenone, 2-hydroxy-4-methoxy-4'-methylbenzophenone; 2,2'-dihydroxy-4-methoxybenzophenone; homomenthyl salicylate; 2-ethylhexyl 2-hydroxybenzoate; dimethicodiethyl-benzalmalonate; 3-(4-(2,2-bis-ethoxycarbonylvinyl)phenoxy)propenyl)methoxysiloxaneldimethylsiloxane copolymer; 4-(tert-butyl)-4'-methoxydibenzoylmethane; 2-(4'-diethylamino-2'-hydroxybenzoyl)benzoic acid hexyl ester; dioctylbutylamidotriazone (INCI: Diethylhexyl-Butamidotriazone); 2,4-bis[5-1(dimethylpropyl)benzoxazol-2-yl(4-phenyl)imino]-6-(2-ethylhexyl)imino-1,3,5-triazine with the CAS No. 288254-16-0; 2,4-bis{[4-(2-ethylhexyloxy)-2-hydrcxy]phenyl}-6-(4-methoxypheny])-1,3,5-triazine (INCI: Bis-Ethyl-hexyloxyphenol Methoxyphenyl Triazine); 4,4',4"-(1,3,5-triazine-2,4,6-triyltriimino)trisbenzoic acid tris(2-ethylhexyl) ester (also: 2,4,6-tris[anilino(p-carbo-2'-ethyl-1'-hexyloxy)]-1,3,5-triazine (INCI: Ethylhexyl Triazone); 2,4,6-tri-biphenyl-4-yl-1,3,5-triazine; merocyanines.

2. Cosmetic emulsion according to Claim 1, **characterized in that** the preparation comprises xanthan gum.

3. Cosmetic emulsion according to one of the preceding claims, **characterized in that** the preparation comprises polyglyceryl-3 methylglucose distearate in a concentration of from 0.05 to 5% by weight, based on the total weight of the preparation.

4. Cosmetic emulsion according to one of the preceding claims, **characterized in that** the preparation comprises silica dimethyl silylate in a concentration of from 0.05 to 5% by weight, based on the total weight of the preparation.

5. Cosmetic emulsion according to one of the preceding claims, **characterized in that** the preparation comprises acrylates/C10-30 alkyl acrylates crosspolymer, in a concentration of from 0.05 to 1.5% by weight, based on the total weight of the preparation.

6. Cosmetic emulsion according to one of the preceding claims, **characterized in that** the preparation comprises xanthan gum in a concentration of from 0.05 to 0.5% by weight, based on the total weight of the preparation.

7. Cosmetic emulsion according to one of the preceding claims, **characterized in that** the preparation comprises one or more active ingredients selected from the group of the compounds glycyrrhetic acid, urea, arctiin, alpha-lipoic acid, folic acid, phytoene, D-biotin, coenzyme Q10, alpha-glucosylrutin, carnitine, carnosine, caffeine, natural and/or synthetic isoflavonoids, glycerylglucose, creatine, creatinine, taurine, tocopherol, tocopherol acetate, β-alanine and/or licochalcone A.

8. Cosmetic emulsion according to one of the preceding claims, **characterized in that** the preparation comprises propylene glycol, butylene glycol, 2-methylpropane-1,3-diol, 1,2-pentanediol, 1,2-hexanediol, 1,2-octanediol and/or 1,2-decanediol.

9. Cosmetic emulsion according to one of the preceding claims, **characterized in that** the preparation comprises phenoxyethanol and/or methylisothiazolinone.

10. Cosmetic emulsion according to one of the preceding claims, **characterized in that** the preparation has a viscosity of from 200 to 2500 mPas at a temperature of 25°C measured using Rheomat 123 from proRheo with spindle 1 (measurement range 300-10 000 mPas).

11. Pump dispenser comprising an emulsion according to one of Claims 1 to 12

12. Aerosol can comprising an emulsion according to one of Claims 1 to 12.

## Revendications

1. Émulsion cosmétique contenant
a) du Polyglyceryl-3-Methylglucose Distearate,
b) du Silica Dimethyl Silylate et
c) de l'Acrylates/C 10-30 Alkylacrylates Crosspolymer,
**caractérisée en ce que** la préparation contient un ou plusieurs filtres UV choisis dans le groupe des composés acide 2-phénylbenzimidazole-5-sulfonique et/ou ses sels ; sels d'acide phénylène-1,4-bis-(2-benzimidazyl)-3,3'-5,5'-tétrasulfonique ; 1,4-di(2-oxo-10-sulfo-3-bornylidènemathyl)-benzène et ses sels ; sels d'acide 4-(2-oxo-3-bornylidèneméthyl)benzènesulfonique ; sels d'acide 2-méthyl-5-(2-oxo-3-barnylidèneméthyl)sulfonique ; 2,2'-méthylène-bis(6-(2H-benzotriazol-2-yl)-4-(1,1,3,3-tétraméthylbutyl)-phénol) ; 2-(2H-benzotriazol-2-yl)-4-méthyl-8-[2-méthyl-3-[1,3,3,3-tétraméthyl-1-[(triméthylsilyl)oxy]disiloxanyl]propyl]-phénol ; 3-(4-méthylbenzylidène)camphre ; 3-benzylidènecamphre ; salicylate d'éthylhexyle ; acide téréphtalidènedicamphosulfonique ; 2-cyano-3,3-diphénylacrylate de 2-éthylhexyle ; 4-(diméthylamino)benzoate de 2-éthylhexyle ; 4-(diméthylamino)benzoate d'amyle ; 4-méthoxybenzalmalonate de di(2-éthylhexyle) ; 4-méthoxycinnamate de 2-éthylhexyle ; 4-méthoxycinnamate d'isoamyle ; 2-hydroxy-4-méthoxybenzophénone, 2-hydroxy-4-méthoxy-4'-méthylbenzophénone ; 2,2'-dihydroxy-4-méthoxybenzophénone ; salicylate d'homomenthyle ; 2-hydroxybenzoate de 2-éthylhexyle ; benzalmalonate de diméthicodiéthyle ; copolymère 3-(4-(2,2-biséthoxycarbonylvinyl)-phénoxy)propényl)-méthoxysiloxane / diméthylsiloxane ; 4-(tert-butyl)-4'-méthoxydibenzoylméthane ; 2-(4'-(diéthylamino-2'-hydroxybenzoyl)benzoate d'hexyle ; dioctylbutylamidotriazone (INCI : Diethylhexyl-Butamidotriazone) ; 2,4-bis-[5-1(diméthylpropyl)benzoxazol-2-yl-(4-phényl)-imino]-6-(2-éthylhexyl)-imino-1,3,5-triazine portant le n° CAS 288254-16-0 ; 2,4-bis-{[4-(2-éthyl-hexyloxy)-2-hydroxy]-phényl}-6-(4-méthoxyphényl)-1,3,5-triazine (INCI : Bis-Ethylhexyloxyphenol Methoxyphenyl Triazin) ; 4,4',4"-(1,3,5-triazine-2,4,6-triyltriimino)-tris-benzoate de tris(2-éthylhexyle) (également: 2,4,6-tris[anilino-(p-carbo-2'-éthyl-1'-hexyloxy)]-1,3,5-triazine (INCI : Ethylhéxyl Triazone) ; 2,4,6-tribiphényl-4-yl-1,3,5-triazine ; mérocyanines.

2. Émulsion cosmétique selon la revendication 1, **caractérisée en ce que** la préparation contient de la gomme xanthane.

3. Émulsion cosmétique selon l'une quelconque des revendications précédentes, **caractérisée en ce que** la préparation contient du Polyglyceryl-3 Methylglucose Distearate à une concentration de 0,05 à 5 % en poids, par rapport au poids total de la préparation.

4. Émulsion cosmétique selon l'une quelconque des revendications précédentes, **caractérisée en ce que** la préparation contient du Silica Dimethyl Silylate à une concentration de 0,05 à 5 % en poids, par rapport au poids total de la préparation.

5. Émulsion cosmétique selon l'une quelconque des revendications précédentes, **caractérisée en ce que** la préparation contient de l'Acrylates/C10-30 Alkylacrylates Crosspolymer à une concentration de 0,05 à 1,5 % en poids, par rapport au poids total de la préparation.

6. Émulsion cosmétique selon l'une quelconque des revendications précédentes, **caractérisée en ce que** la préparation contient de la gomme xanthane à une concentration de 0,05 à 0,5 % en poids, par rapport au poids total de la préparation.

7. Émulsion cosmétique selon l'une quelconque des revendications précédentes, **caractérisée en ce que** la préparation contient une ou plusieurs substances actives choisies dans le groupe des composés acide glycyrrhétinique, urée, arctiine, acide alpha-lipoïque, acide folique, phytoène, D-biotine, coenzyme Q10, alpha-glucosylrutine, carnitine, carnosine, caféine, isoflavonoïdes naturels et/ou synthétiques, glycérylglucose, créatine, créatinine, taurine, tocophérol, acétate de tocophérol, β-alanine et/ou licochalcone A.

8. Émulsion cosmétique selon l'une quelconque des revendications précédentes, **caractérisée en ce que** la préparation contient du propylèneglycol, du butylèneglycol, du 2-méthylpropane-1,3-diol, du 1,2-pentanediol, du 1,2-hexanediol, du 1,2-octanediol et/ou du 1,2-décanediol.

9. Émulsion cosmétique selon l'une quelconque des revendications précédentes, **caractérisée en ce que** la préparation contient du phénoxyéthanol et/ou de la méthylisothiazolinone.

10. Émulsion cosmétique selon l'une quelconque des revendications précédentes, **caractérisée en ce que** la préparation présente à une température de 25 °C une viscosité de 200 à 2 500 mPa.s, mesurée à l'aide du Rheomat 120 de proRheo avec la broche 1 (plage de mesure 300 - 10 000 mPa.s).

11. Distributeur à pompe contenant une émulsion selon l'une quelconque des revendications 1 à 12.

12. Récipient aérosol contenant une émulsion selon l'une quelconque des revendications 1 à 12.
